(19) **Europäisches Patentamt / European Patent Office / Office européen des brevets**

(11) **EP 4 076 192 B1**

(12) # EUROPEAN PATENT SPECIFICATION

(45) Date of publication and mention of the grant of the patent:
**31.01.2024 Bulletin 2024/05**

(21) Application number: **20811135.1**

(22) Date of filing: **16.10.2020**

(51) International Patent Classification (IPC):
*A61B 5/20* (2006.01)   *A61B 5/00* (2006.01)
*G01F 3/00* (2006.01)   *G01F 1/50* (2006.01)
*G01F 23/14* (2006.01)   *G01F 23/80* (2022.01)
*G01F 23/20* (2006.01)   *G16H 50/20* (2018.01)
*G01F 9/00* (2006.01)

(52) Cooperative Patent Classification (CPC):
**A61B 5/208; A61B 5/7207; A61B 5/7221;**
**G01F 9/003; G01F 23/20; G01F 23/80;**
**G16H 50/20;** A61B 2505/03; A61B 2505/07;
A61B 2562/0247; A61B 2562/0252

(86) International application number:
**PCT/US2020/056103**

(87) International publication number:
**WO 2021/126352 (24.06.2021 Gazette 2021/25)**

(54) **UROFLOWMETRY SIGNAL ARTIFACT DETECTION AND REMOVAL SYSTEMS AND METHODS**

SYSTEME UND VERFAHREN ZUR DETEKTION UND ENTFERNUNG VON HARNFLUSSMESSSIGNALARTEFAKTEN

SYSTÈMES ET PROCÉDÉS DE DÉTECTION ET D'ÉLIMINATION D'ARTEFACTS DE SIGNAL DE DÉBITMÉTRIE URINAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.12.2019 US 201962948804 P**
**14.10.2020 US 202017070858**

(43) Date of publication of application:
**26.10.2022 Bulletin 2022/43**

(73) Proprietor: **Laborie Medical Technologies Corp.**
**Williston, Vermont 05495 (US)**

(72) Inventors:
• **DACKO, Adrian, G.**
**Mississauga, Ontario L5H4G3 (CA)**

• **COLE, David, Nathaniel**
**Brampton, Ontario L6Z 4E4 (CA)**
• **MAUNDER, Simon, B.**
**Leeds LS8 2HA (GB)**
• **ZHANG, Hance**
**Milton, Ontario L9T 7N9 (CA)**
• **DRIVER, Christopher**
**Oakville, Ontario L6J 6W7 (CA)**

(74) Representative: **Howe, Steven**
**Reddie & Grose LLP**
**The White Chapel Building**
**10 Whitechapel High Street**
**London E1 8QS (GB)**

(56) References cited:
**EP-A1- 3 412 206      WO-A1-2013/021207**
**US-A1- 2006 235 353      US-A1- 2010 152 684**

EP 4 076 192 B1

**Description**

**BACKGROUND**

Uroflowmetric tests generally measure the flow of urine. Uroflowmetry may track many aspects of a patient voiding, such as tracking how fast urine flows, how much urine flows out, and how long it takes a patient to fully void. By measuring the average and top rates of urine flow, uroflowmetry tests can show various health concerns regarding your urinary tract. However, there is always a need to provide more accurate data to a physician to better diagnosis health concerns.

US 2006/235353 A1 discloses a patient hydration system and method wherein hydration fluid is administered to a patient and the patient's urine output is measured either by weight or some other suitable way. A controller is responsive to the patient's urine output measurement and configured to control the infusion rate to hydrate the patient based on the patient's urine output. The controller also detects abnormal patient urine output measurements and initiates corrective action in response.

**SUMMARY**

The invention is set out in the independent claims, to which reference should now be made. Additional, optional features are provided in the dependent claims.

**BRIEF DESCRIPTION OF THE DRAWINGS**

FIG. 1 illustrates an exemplary uroflowmetry system.
FIGS. 2A and 2B provide two exemplary artifacts, a positive form artifact and a negative artifact.
FIG. 3 provides an exemplary flow chart of a noise artifact detection method which may be followed to identify artifacts.
FIGS. 4A and 4B illustrate the exemplary artifacts of FIGS. 2A and 2B prior to normalization.
FIG. 5 provides an example of volume data comprising a leak.
FIG. 6 provides a table comprising various parameters which may be configured when analyzing artifacts and/or artifact complexes.
FIGS. 7A and 7B provide exemplary maximum durations for two exemplary artifacts, a positive form artifact and a negative form artifact.
FIG. 8 provides an example showing the relationship between a mean, the mean squared error, and the volume samples.
FIG. 9 details an exemplary relationship between the cluster median, cluster size, and the volume samples.
FIG. 10 provides some exemplary values when performing baseline checks and/or determining whether or not an interval between two baselines is a flow, a leak, noise, or an artifact.

**DETAILED DESCRIPTION**

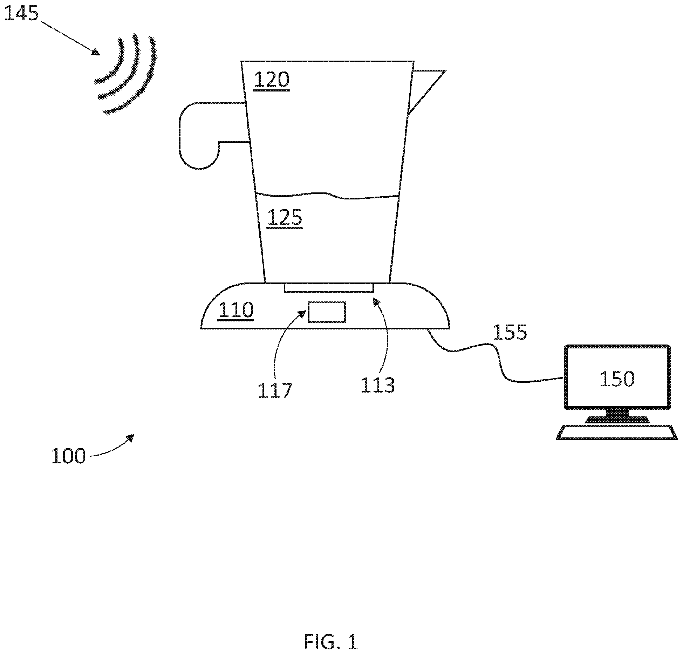

FIG. 1 provides an exemplary uroflowmetry system 100 which may be used to analyze uroflowmetry data as discussed herein. according to the invention, the uroflowmetry system 100 comprises a uroflowmeter 110 to measure pressure waves to determine various attributes a voiding. However in some circumstances, pressure waves external to uroflowmeter 110 (e.g. external event 145) may produce noise and/or artifacts in uroflowmeter data. An external event 145 may comprise external vibrations or noises that cause disruptions or movement on or near a sensor in the uroflowmeter 110 (e.g. uroflowmeter sensor 113) resulting in the noise and/or artifact from the external event 145 to be present in the data (e.g. volume data and/or flow rate data). In some embodiments, the uroflowmeter 110 may be configured to measure the level of liquid 125 in a container 120 (e.g. a beaker, a cup, or the like). In such embodiments, the external event 145 (e.g. the movement of the container 120) may cause artifacts to be present in the data, such as if the container 120 is kicked, spilled, or bumped.

As discussed herein, the uroflowmeter 110 comprises uroflowmeter sensor 113. Uroflowmeter sensor 113 may comprise a variety of sensor types, such as a load cell, a transducer, or the like. In such examples, the external events 145 may momentarily cause a higher or lower pressure applied to the uroflowmeter sensor 113, affecting the pressure inside the uroflowmeter pressing down on the uroflowmeter sensor 113. The difference in pressure may then result in an artifact being present in the data.

As discussed herein, a uroflowmeter comprises a sensor (e.g. a load cell, a transducer, or the like). In such examples, the external events may momentarily cause a high or lower pressure applied to the uroflowmeter sensor, affecting the pressure inside the uroflowmeter pressing down on the sensor. The difference in pressure may then result an artifact being present in the data.

Furthermore, in some embodiments the uroflowmeter 110 may be covered or partially covered by a silicon boot or other material to make the system water-proof or more water-resistant. In such examples, there may be a localized pressure difference between the inside and outside of the silicon boot. Accordingly, when external pressures are present the silicon boot may act as a diaphragm which results in additional force being applied to a pressure measurement device (e.g. uroflowmeter sensor 113). Accordingly, the uroflowmeter can have an increased likelihood of detecting pressure waves resulting from an external event 145 which can appear as an artifact or noise in the data.

The external events 145 may come from a variety of sources. For example, an external event 145 may be from a door opening/closing, an HVAC system running, footsteps, mechanical vibrations (e.g. from heavy machinery), movement of the container 120 or portions of the uroflowmeter, or other situations which may emit substantial vibrations and/or noises.

In some embodiments, the data gathered by the uroflowmeter 110 is filtered, such as low pass filtered or bandpass filtered, to reduce and/or eliminate noise from environmental and/or external conditions. In such examples, a 5 Hz lowpass filter can be used because uroflowmeter signals related to physiological sources (e.g. voiding) generally have frequencies of less than 5 Hz, or in some examples less than 1.5 Hz. In some embodiments, the target band can be about 0-1 Hz, 0-1.5 Hz, 0.4-0.8 Hz, or other target bands known to one of ordinary skill in the art. However in some examples, some noise artifacts may have a frequency of less than 5 Hz, such as artifacts from external events 145, and therefore cannot be simply eliminated by lowpass or bandpass filtering without also filtering out important physiological information.

Furthermore, as more data is being gathered from the uroflowmeter 110, such as by collecting from an increased bandwidth collection, the uroflowmeter 110 can collect more external events 145 in the data. Such artifacts in the data may cause the data to include non-uroflowmetry information, be harder to read, and potentially lead to a user (e.g. a physician) interpreting inaccurate results.

To overcome such a problem, a noise artifact detection method may be used as a technical artifact detector which may operate on volume channel data from uroflowmeter 110. The noise artifact detection method can be used to identify localized atmospheric pressure artifacts, vibration artifacts, or the like related to external events 145 near a sensitive measuring device (e.g. uroflowmeter 110) such as the opening and closing of doors. Additionally, the noise artifact detection method as discussed herein may be performed using a noise artifact detection system, such as a system incorporated into uroflowmetry system 100 as shown in FIG. 1.

The noise artifact detection method may be performed in the uroflowmeter 110. Additionally or alternatively the noise artifact detection method may be performed on a separate device, such as an external computation device 150 (e.g. a computer, tablet, smartphone, or the like). In such embodiments, the uroflowmeter 110 may be in communication with the external computation device 150, such as via connection 155. Connection 155 may comprise a variety of connections know in the art, such as a wired connection, a wireless connection, a combination thereof, or the like.

The noise artifact detection method may be used to analyze the morphology of affected waveform shapes in the data produced by uroflowmeter sensor 113. More specifically, the noise artifact detection method may be used to identify artifacts from various external events 145 in the data. In some examples, voiding patterns will not be identified as artifacts. However, other external events, such as taping, splashing, footsteps on the floor, or the like may be identified as artifacts based on various parameters, such as amplitude, frequency, and/or shape of the signal detected by the uroflowmeter 110. When a portion of the data is identified as an artifact, said portion of the data may be removed, interpolated, marked, or the like. For example, a FlowNoiseDataRetraction Event may be initiated and/or generated for the timespan of the artifact, as discussed herein.

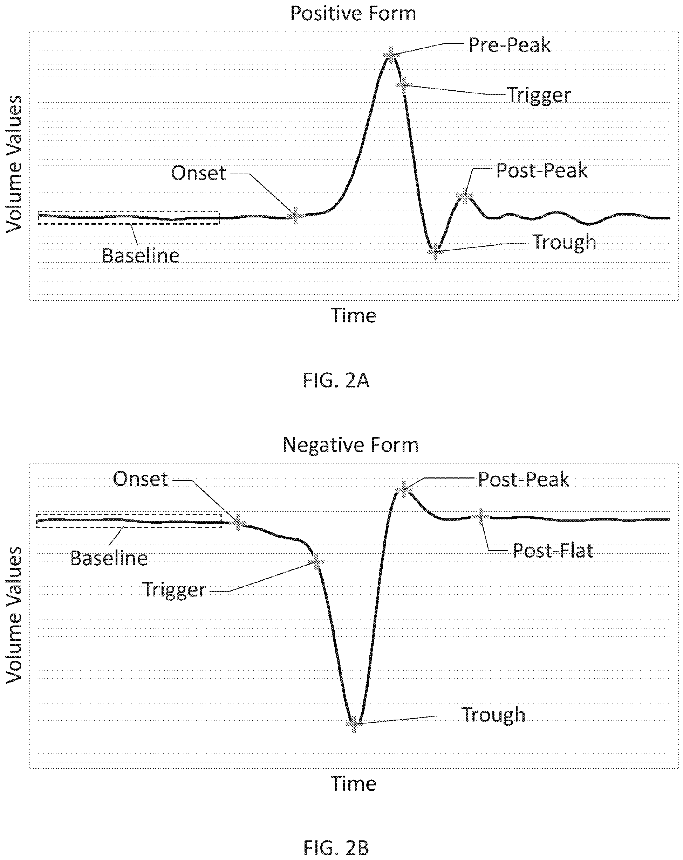

As discussed herein, various artifacts may be identified. In some embodiments, artifacts may come in distinct forms. FIGS. 2A and 2B provide two exemplary artifacts, a positive form artifact and a negative artifact. As shown, FIGS. 2A and 2B provide the relationship between time and change in volume for an exemplary positive form artifact and an exemplary negative form artifact, respectfully. In some embodiments, the positive form artifact shown in FIG. 2A may be the result of a door opening and similarly, the negative form artifact shown in FIG. 2B may be the result of a door closing. However, such artifacts (e.g. positive form, negative form, or the like) may be result of various external events, as discussed herein.

A positive form artifact as shown in FIG. 2A can be characterized by a fast rise in amplitude followed by a trough and a return to a baseline value. Conversely, a negative form artifact as shown in FIG. 2B can be characterized by a fast decrease in amplitude followed by a return to a baseline value.

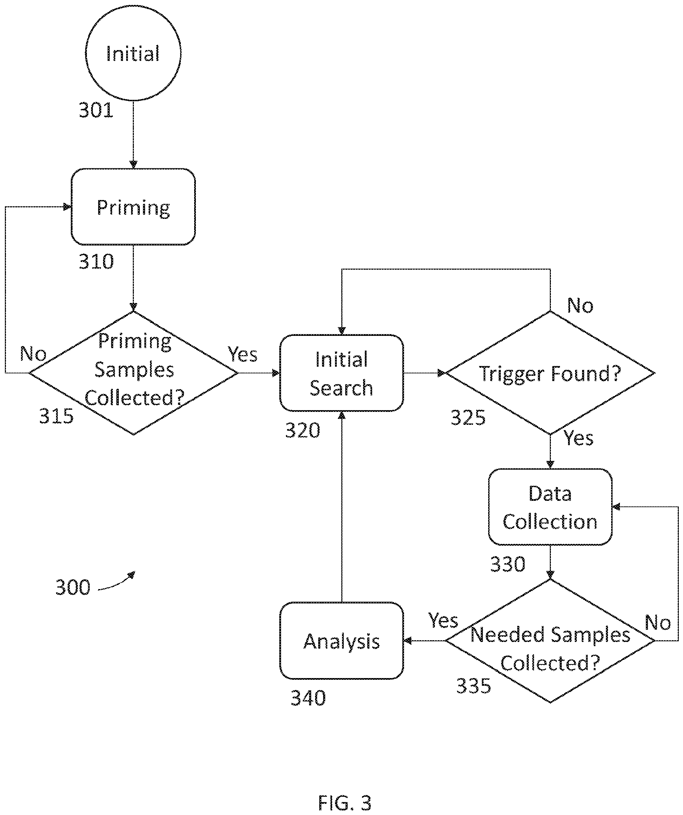

Fig. 3 provides an exemplary flow chart of a noise artifact detection method 300 which may be followed to identify artifacts. As shown, the method 300 may comprise a priming step 310, an initial search step 320, a data collection step 330, and an analysis step 340. With respect to priming step 310, an initialization step 301 may occur prior to the priming step 310, and a check 315 to make sure priming samples have been collected may happen after the priming step 310. Once it is determined that priming samples have been collected (e.g. YES in check 315), the initial search step 320 may be performed.

Additionally, a check 325 for a trigger value may be performed, wherein if no trigger is found (e.g. NO in check 325) then the method reverts back to the initial search step 320 and if a trigger is found (e.g. YES n check 325) then the data collection step 330 may be initialized. In some embodiments, an initial trigger may start again immediately. However, in other embodiments, an initial rigger may happen periodically, or based on other factors (e.g. user input, flow rate, other sensor information, etc.). After data collection in step 330 is initialized, a check 335 to see if the needed amount of samples has been collected. In some embodiments, the samples may be collected at a rate of 100 samples a second, however other sampling rates, such as rates above or below 100 samples a second have been contemplated. In some embodiments, the data collected in step 330 may be placed in a buffer, such as a circular buffer or the like. The needed amount of samples can be an amount of samples, samples spanning an amount of time, or the like. In some embodiments, the samples needed may be based on a predetermined amount. After the needed samples have been collected (e.g. YES in check 335) the analysis step 340 may be performed. Then in some embodiments, another initial search may be performed after analysis is complete. In some embodiments, an initial search may be performed simultaneously with analysis. Various other embodiments similar to those described with respect to FIG. 3 have been contemplated. For example, after the analysis step 340, the process may revert back to the priming step 310 or the initialization step 301 rather than the initial search step 320.

In some embodiments, samples (e.g. volume samples from uroflowmeter sensor 113) are put through a bandpass filter after being received and before being analyzed such as by method 300. Additionally or alternatively, the bandpass filtering may be performed at various other times, such as during the priming step 310, the initial search 320 and/or during the data collection step 330. In some embodiments, every data sample used in during the analysis step 340 may be filtered (e.g. bandpass filtered) prior to the analysis step 340. The bandpass filter may be used to remove frequencies outside of the major power center of a targeted artifact as well as partially normalize the data with respect to concurrent flow. Various bandpass filters may be used, such as ones with various orders as well as ranges. In some examples, a $64^{th}$ order bandpass filter with a finite impulse response (FIR) of 0.4 Hz to 0.8 Hz may be used. In some embodiments, the normalizing the data may comprise normalizing the amplitude against an estimate flow pattern based on the volume data before and after the area under consideration. In some instances, this estimation may be based on a fit line, however other models may be used such as higher order polynomials, exponential models, logarithmic models, or the like.

After samples go through the bandpass filter, depending on which step shown in method 300 is currently being performed, the processing may continue with the processed values. For example, if the method is currently performing the data collection step 330, the process may continue to the analysis step 340.

With respect to FIG. 3, in some embodiments the priming step 310 does not include performing analysis on the data. In such embodiments, the priming step 310 collects samples until a priming duration has been met. In some examples, the priming duration may be a predetermined amount of time, acquiring a predetermined amount of samples, or the like. In some examples, the priming duration may be selectable by a user, such as by a user interface located on the uroflowmeter (e.g. user interface 117) and/or a user interface located on an external device (external computation device 150). Additionally or alternatively, the priming duration may be automatically selected. Furthermore, as shown in FIG. 2, once the priming duration is met (e.g. YES in step 315), the process may move forward to the initial searching step 320.

In some embodiments, the initial search step 320 may comprise a method which determines the various trends of data received from a uroflowmeter (e.g. volume data). This may comprise an algorithm to calculate a current slope in the data. In such embodiments, the slope may be calculated using a best fit model, such as a least-squares best fit model.

In some embodiments, if the calculated slope is above or below a trigger threshold (e.g. YES in check 325), the method may record the collected sample, such as by a sample stamp. In some embodiments, the trigger threshold may be when the calculated slope is at or below 0 mL/s. When the calculated slope falls below 0 mL/s artifact detection can be initiated because the overall volume should not decrease during a uroflowmetry test or the like. Additionally, the trigger threshold may be other values, such as values above or below 0 mL/s, such as -1.2 mL/s. In some embodiments, the threshold may be a value at or less than 0 mL/s. Furthermore, trigger thresholds may be set based on the patient, such as age gender, weight, medical conditions, or other conditions known to one of ordinary skill in the art. In some embodiments, the threshold detection is after an initial lowpass and/or bandpass filter is used to reduce the number of false artifacts.

When the trigger threshold is met (e.g. YES in check 325), the method may proceed to the data collection step 330. In some embodiments, the data collection step may comprise collecting all necessary samples prior to performing the analysis step 340. In some examples, data can be collected for a given amount of time, such as 15000ms after a trigger threshold is found, such as starting from the sample stamp as discussed herein. However, other amounts of time, such as more than 15000ms and less than 15000ms have been contemplated. Alternatively, data may be collected until a specified amount of samples have been collected. In some embodiments, data may be continually collected and stored in a buffer, such as a rolling buffer. In such embodiments, the rolling buffer may collect data over a predetermined period of time (e.g. 15000ms), for a predetermined amount of data samples, or the like.

Once the samples have been collected (e.g. YES in check 335) the method may continue to the analysis step 340. In some embodiments, the analysis step 340 may be performed simultaneously to the data collection step 330 and/or the samples may be stored for later use.

As described herein, the analysis step may be performed to determine whether or not an artifact is present after an event has occurred (e.g. a trigger threshold has been reached). In some embodiments, the analysis step 340 may comprise determining whether or not the morphology of the potential artifact represents or closely represents the morphology of a known artifact, such as a positive form artifact (e.g. opening a door) and a negative form artifact (e.g. closing a door) as shown in FIGS. 2A and 2B. However, morphologies of other artifacts may also be used. Additionally, the analysis step 340 may take place after an initial filtering of the data, such as with a lowpass filter or a bandpass filter as discussed herein.

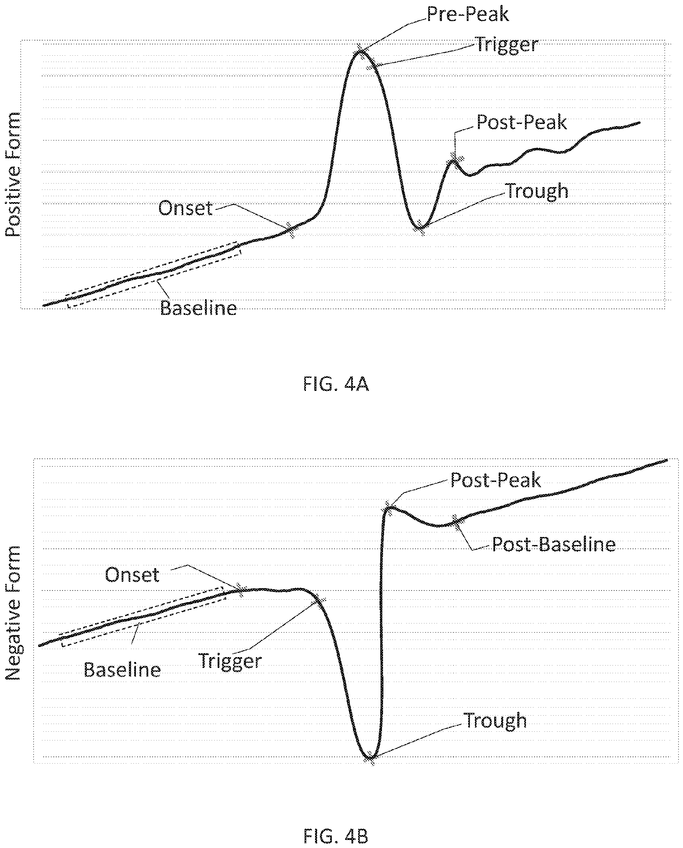

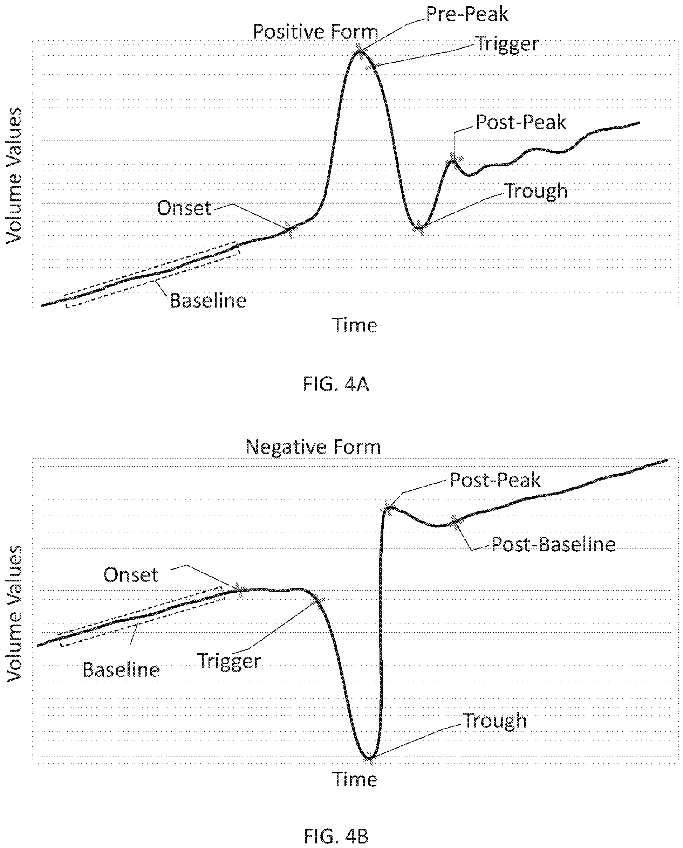

With respect to FIGS. 2A and 2B, the general trend of the volume data is shown to be flat, such as during times where there is little to no change in overall volume (e.g. the patient is not voiding). However, data may also be collected during times when there is an overall change in volume (e.g. the patient is voiding). In embodiments wherein the patient is voiding, the baseline may be adjusted and/or compensated by using a best fit model (e.g. least-squares best fit model or the like) as discussed above. In such embodiments, the data may be normalized relative to an initial baseline value and/or the closest baseline prior to a triggering event. FIGS. 4A and 4B provide an illustration of FIGS. 2A and 2B prior to a normalization event. As shown, both FIGS. 4A and 4B have a general trend of the volume data increasing with a positive slope, but the general form of each artifact (e.g. positive form artifact and negative form artifact) may still be present. In some embodiments, the data may be normalized, such as shown in FIG. 2A and 2B.

In some examples, the best fit may be subtracted from the volume data in order to provide a more relatively flat dataset as shown in FIGS. 2A and 2B as well as discussed herein. Such adjustments may be beneficial when interpreting/analyzing the data, as the flow rate when a patient is voiding isn't always consistent, so adjusting the data may provide a more accurate artifact detection. Even though such a normalization is not always necessary, for ease of discussion, embodiments herein are described with respect to the normalization of the data.

Turning back to FIG. 3, in some embodiments the analysis step 340 may happen in real time, such as while sample data is being collected (e.g. data collection step 330) or shortly after (e.g. 1/10$^{th}$ of a second, a predetermined amount of samples, as soon as data is gathered in the buffer, as soon as a threshold is detected, etc.). Alternatively, the analysis step 340 may happen after data collection, such as after a predetermined amount of time after a threshold, upon user input, or after all samples have been collected.

In some embodiments, method 300 may look for a particular event prior to determining whether or not an artifact complex is present (e.g. performing analysis step 340). As described herein, the particular event may be the flow rate going below a threshold value, such as 0 mL/s, -1.2 mL/s, or the like.

During the analysis step, the method may look for a few based elements to help determine which form the artifact is in as well as the start and/or end of the artifact complex. The basic elements may comprise one or more of an initial baseline, trough, post-peak, pre-peak, and post-baseline which are described in further detail herein and shown with respect to FIGS. 2A and 2B. In some embodiments, the elements may comprise a plurality of samples, such as a plurality of samples taken between to samples or a group of samples taken between two times. In some embodiments, the based elements may be determined after the data is normalized to an initial baseline, such as shown in FIGS. 2A and 2B.

An initial Baseline can be defined as the area prior to the start of the artifact. In some embodiments, the initial base line is calculated as an arithmetic mean (e.g. average) of a window, or grouping, of the process sample values before the trigger. Alternatively, the initial baseline may use other methods to calculate its value, such as a best fit line from least means squared, an R2 value, or the like.

A trough can be defined as the lowest local minima in the window, or group of samples, bounded by the trigger and a post-baseline. In no local minima can be determined or found, then the trough can be defined as the most-flat time bounded by the trigger and the post-baseline.

A post-peak can be defined as the largest local maxima in the window, or group of samples, bounded by the trigger and the post-baseline.

A pre-peak can be defined as the largest local maxima in the window, or group of samples, bounded by the initial baseline and the trigger.

In some embodiments, an event (e.g. positive form, negative form) may need to have an identified trough to be considered as an artifact. In some examples, the positive form artifact may be identified if the pre-peak is greater than the initial baseline and the post-peak. Additionally or alternatively, the negative form artifact may be identified if the identified trough is after the trigger. As described herein, FIG. 2A illustrates an exemplary positive form artifact and FIG. 2B illustrates an exemplary negative form artifact. When identifying various attributes of the potential artifact (e.g. positive form artifact, negative form artifact, etc.) the data may be normalized based on the initial baseline, as described herein.

If the form is believed to be a positive form, additional elements may be determined. In some examples, the onset can be determined. The onset can be defined as the point where the positive deflection begins prior to the peak (shown in FIG. 2A). Various methods may be used to determine the onset, such as the two described below. In some embodiments, the first method is used and then the second method is used if the first method does not return an accurate onset value.

For example, first method for determining the onset may comprise locating a local minima or flat area in the span prior from the pre-peak that is less than 10% of the pre-peak amplitude from the initial baseline. With respect to FIG. 2A, it can be seen that the onset marked on the positive form is shown as the point which is roughly 10% of the Pre-Peak value. A second method for determining the onset may comprise locating the steepest point in the span prior to the pre-peak and then search for the flattest area prior to the steepest point. In some examples, the flattest area is additionally after the initial baseline.

In some examples, the artifact complex may be rejected as a potential artifact if no onset is identified.

After the onset is identified, the duration of the artifact can be estimated. In some examples, the duration of the artifact may be estimated using the following empirical ratio:

$$\text{EQ. 1: Duration Artifact} = \text{Amplitude Peak-to-Peak} * 40$$

Wherein the Duration Artifact is the duration of the artifact from the onset in milliseconds (ms) and the AmplitudePeak-to-Peak which can be the difference between the peak and the trough within the potential artifact, or the difference between the pre-peak and the trough and can be measured in milliliters (mL). The value of 40 may be based on the used uroflowmeter (e.g. uroflowmeter 110) and the sensor used (e.g. uroflowmeter sensor 113). In some embodiments, a value of above or below 40 may be used to calculate the Duration Artifact depending on the system and/or the surrounding environment.

The estimated duration (e.g. Duration Artifact) calculated using EQ. 1 can be bounded by a maximum estimated duration, as discussed herein. Additionally, an endpoint can be checked against any detected elements; and if any parts of the artifact complex are found outside of the estimated range, that point is set as the endpoint.

Once the bounds are calculated, a normalized peak-to-peak amplitude can be calculated for the entire artifact complex and checked to see if it meets a threshold value. The normalized peak-to-peak amplitude may be calculated based on the normalization of the initial baseline or an additional normalization. In some embodiments, the data may be normalized based on a line between the start and end of the potential artifact (e.g. a line between the initial baseline value and the post-baseline value. In some embodiments, the threshold value may be 0.30 mL, however values above 0.30 mL and below 0.30 mL have been contemplated. Additionally or alternatively, the peak-to-peak amplitude may be based on the data between start and end of the potential artifact.

In some embodiments, potential artifacts can be evaluated to see if the peak positive flow is above a threshold. In such embodiments, the threshold may be between 0.4 mL/s and 0.6 mL/s, such as 0.48mL/s; however values above 0.6 mL/s and below 0.4 mL/s have been contemplated.

After the artifact complex is identified (e.g. as a positive form, negative form, not an artifact, etc.). A holdoff point may be used and then the method may switch back into the initial search step 320. In some embodiments, the holdoff point may be 10ms, however values above 10ms and below 10ms have been contemplated.

If the form is believed to be a negative form, additional elements may be determined. In some examples, the onset and post-baseline may be determined. The onset can be defined as the point where the negative deflection begins in the window prior to the trigger. In some examples, this can be found by looking for the earliest point of negative slope in the window. The post-baseline can be defined as the flattest slope in the area after the post-peak. In some examples, the artifact complex may be rejected as a potential artifact if no onset and post-baseline are identified.

After the onset and the post-baseline are identified, the duration of the artifact can be estimated. In some examples, the duration of the artifact may be estimated using the following empirical ratio:

$$\text{EQ. 2: Duration Artifact} = \text{Amplitude Peak-to-Peak} * 40$$

Wherein Duration Artifact is the duration of the artifact from the onset in milliseconds (ms) and the Amplitude Peak-to-Peak, which can be the difference between the trough and the post-peak or the trough and post-baseline and can be measured in milliliters (mL). In some embodiments, a value of above or below 40 may be used to calculate the Duration Artifact depending on the system and/or the surrounding environment.

As discussed herein, the estimated duration (Duration Artifact) calculated in equation 2 can be bounded by a maximum estimated duration. Additionally, an endpoint can be checked against any detected elements; and if any parts of the artifact complex are found outside of the estimated range, that point is set as the endpoint.

Once the bounds are calculated, a normalized peak-to-peak amplitude can be calculated for the entire artifact complex and checked to see if it meets a threshold value. The normalized peak-to-peak amplitude may be calculated based on the normalization of the initial baseline or an additional normalization. In some embodiments, the data may be normalized based on a line between the start and end of the potential artifact (e.g. a line between the initial baseline value and the post-baseline value. In some embodiments, artifact complex's which do not meet the threshold value are not identified as artifacts. In some embodiments, the threshold value may be 0.08 mL, however values above 0.08 mL and below 0.08 mL have been contemplated.

Additionally or alternatively, potential artifacts can be evaluated to see if the peak positive flow is above a threshold. In such embodiments, the threshold may be between 0.4 mL/s and 0.6 mL/s, such as 0.48mL/s; however values above 0.6 mL/s and below 0.4 mL/s have been contemplated.

After the artifact complex is identified (e.g. as a positive form, negative form, not an artifact, etc.). A holdoff point may be used and then the algorithm may switch back into the initial search state. In some embodiments, the holdoff point may be 10ms, however values above 10ms and below 10ms have been contemplated.

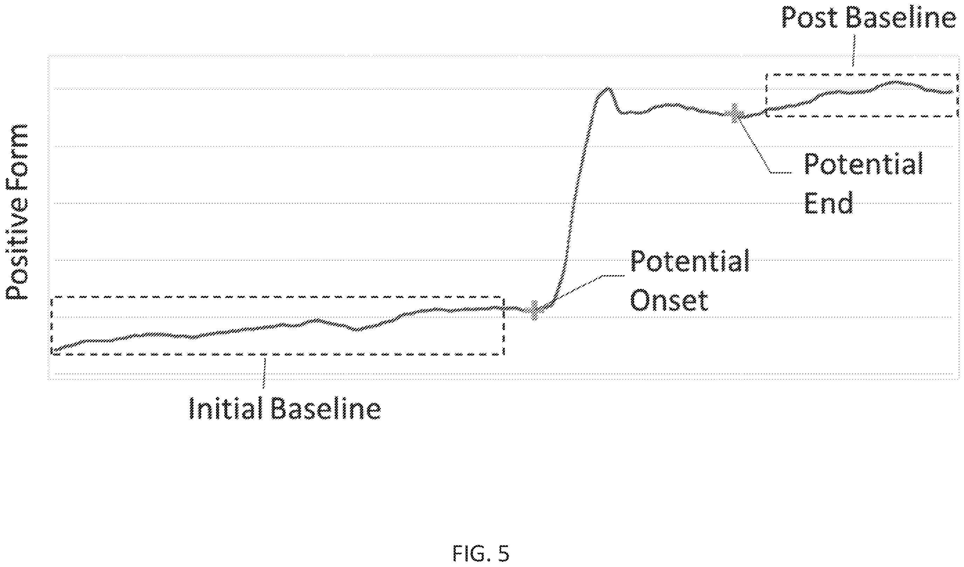

FIG. 5 provides an example of data comprising a leak. A leak may be from a patient voiding unexpectedly or the like. As can be seen, the artifact of a leak can be very similar to the positive form and negative form shown in FIGS. 2A and 2B. However, a leak may contain important diagnostic information and thus shouldn't be removed.

In some embodiments, leak detection is evaluated if the initial baseline and the post-baseline slopes are flat or sufficiently flat, such as shown in FIG. 5. In some embodiments, sufficiently flat may be within a threshold, such as between 1.2mL/s and -1.2mL/s, however other thresholds have been contemplated.

Methods may also include determining and/or calculating a mean value of the baseline areas (e.g. initial-baseline and post-baseline) and may determine a baseline delta based on the differences between the initial baseline and the post-baseline.

In some embodiments, a leak is determined if the baseline delta is greater than a threshold of the normalized Peak-to-Peak amplitude (e.g. Peak-to-Trough amplitude and/or Trough-to-Post-Peak amplitude). In such examples, if the delta baseline is above a certain value, the event is determined to be a leak rather than an artifact from an external event (e.g. positive form, negative form, or the like). For example, the threshold may be 15% of the Peak-to-Trough amplitude and/or Trough-to-Post-Peak amplitude, however other values higher than 15% and lower than 15% have been contemplated. In some embodiments, when the overall volume does not change substantially, or in relation to the size of the artifact, the artifact may be determined to be an artifact from an external event (e.g. positive form, negative form, or the like) rather than a physiological event (e.g., leak, initialization of voiding). Accordingly, in some embodiments a comparison is made between the volume before and after the event and the change in volume is compared to, for instance, the size of the event to determine if the event is something other than a physiological event. Such analysis may be used to trigger the analysis of such an event to determine if a noise artifact is present. Noise artifacts in the data may be identified via many different methods, such as those disclosed herein, as well as other types of known signal analysis and comparisons to atlases of known artifacts and via many types of analyses including via training artificial intelligence to recognize many different noise artifacts.

Additionally or alternatively, if the baseline delta is negative (e.g. the initial baseline is less than the post-baseline) it may be determined that an error has occurred, the sensor (e.g. uroflowmeter sensor 113) is mis-calibrated, or the like. However, in some situations, the baseline delta may have a negative value, such as if the container (e.g. container 120) is bumped and a portion of the liquid within the container is spilled. In such examples, the baseline delta may reflect the loss of liquid within the container. In some embodiments, the post-baseline value may be checked with respect to the trough value and/or the local minimum between the initial baseline and the post-baseline. In such embodiments, if the post-baseline value is less than the trough value and/or minimum value it may be determined that an error has occurred, the sensor is mis-calibrated, or the like.

In some embodiments as discussed herein, there is a holdoff period between trigger point searches (e.g. check 325). However, there is a possibility that a suitable waveform morphology can be found that has an onset within the window of a prior detected artifact complex. In such examples, the subsequent artifact complex may have the onset adjusted such that it occurs only after the holdoff window.

Additionally or alternatively, a leak may be determined based on whether the event fits into one or more predetermined cases. For example, first case may be identifying physiological leaks with a very low flow, a second case may be identifying physiological leaks which are quick/short, and a third case may be identifying artifacts during a flow which may look similar to a quick/short physiological leak.

With respect to the first case, in some situations a physiological leak may be very low, or small however it may still be important to count as a physiological leak rather than an artifact. Identifying such physiological leaks may comprise determining if the flow rate before and after an event is less than a threshold flow rate (e.g. less than or equal to 0.1 mL/s or like). Additionally, identifying such physiological leaks may comprise determining if the post-baseline value goes up by more than a threshold percent of the peak value (e.g. 40% or the like). And if the threshold percent is reached, the event may be determined as a physiological leak rather than an artifact.

With respect to the second case, in some situations a physiological leak may not last long (e.g. be relatively short or quick), however it may still be important to identify as a physiological leak rather than an artifact. Identifying such physiological leaks may comprise determining if the flow rate before and after an event is above a threshold flow rate (e.g. being greater than 0.8 mL/s or the like). Additionally, identifying such physiological leaks may comprise comparing the peak value to the post-baseline value, and if the post-baseline value goes up by less than a threshold percent of the peak value (e.g. 50% or the like), the event may be identified as a physiological leak during flow rather than an artifact.

With respect to the third case, in some situations various artifacts may look very similar to physiological leaks as discussed above with respect to the second case, however it may still be important to count as an artifact rather than a genuine physiological leak. In such situations, to identifying such artifacts may comprise determining if the flow rate before and after an event is below a threshold flow rate, which in some examples may be complementary to the threshold flow rate discussed herein with respect to the third case (e.g. being less than 0.8 mL/s). Additionally, the peak value may be compared to the post-baseline value, and if the post-baseline value goes up by more than a threshold percent of the peak value, the event may be identified as an artifact. In some embodiments, the threshold percent may be complementary to the threshold percent discussed herein with respect to the second case (e.g. greater than 50%).

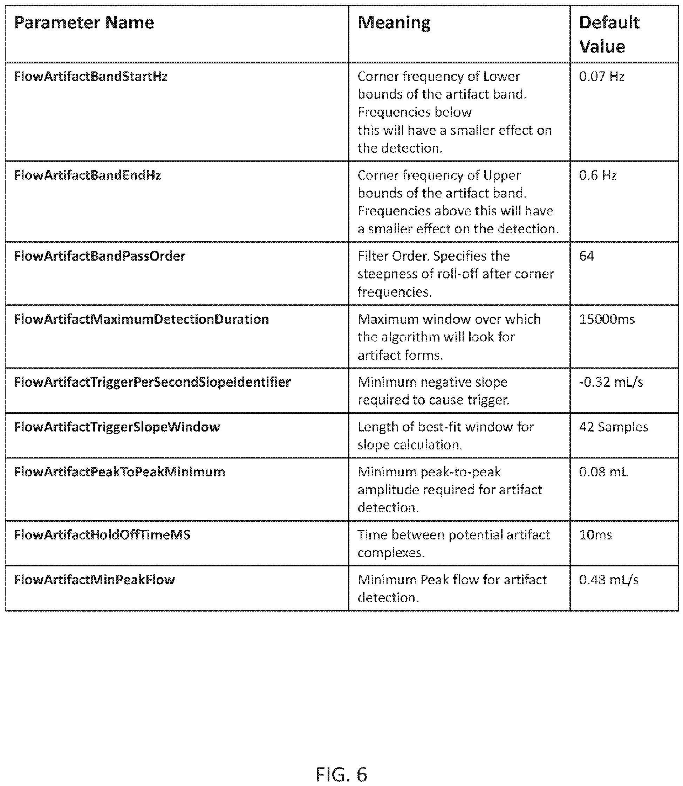

FIG. 6 provides a table comprising various parameters which may be configured when analyzing artifacts and/or artifact complexes. Each parameter detailed in FIG. 6 has an exemplary detailed value, however as described herein, the default value is meant to be exemplary rather than limiting. Other values, such as values above and below the default value.

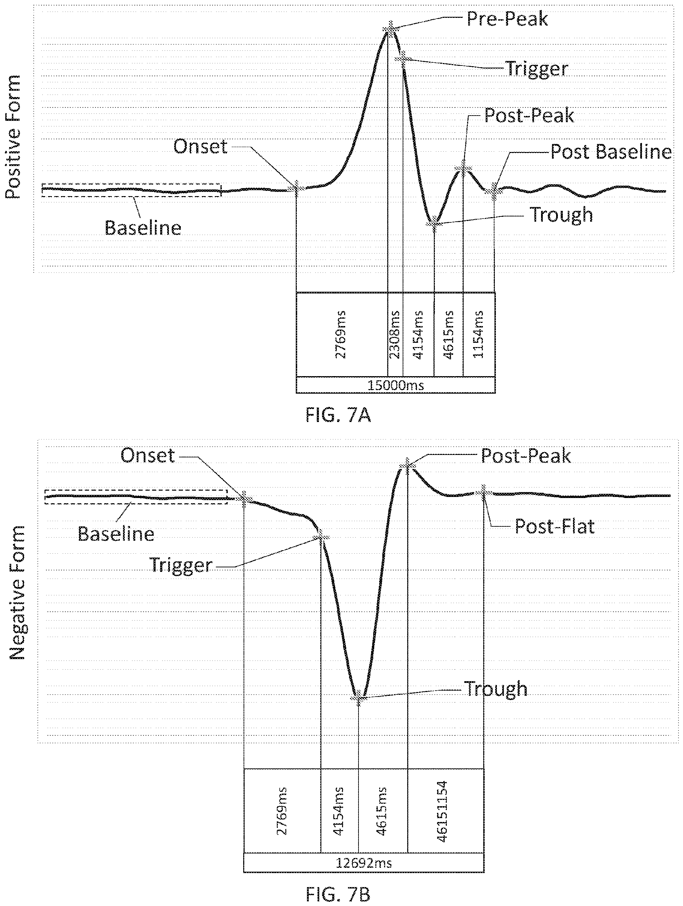

In some embodiments, a maximum artifact duration may be defined. For example, the maximum artifact duration may be 15000ms, however durations less than 15000ms or more than 15000ms have been contemplated. For example, the maximum artifact duration may be adjusted for a variety of qualities, such as sensor type, uroflowmeter type, location, temperature, air pressure. In some embodiments, as the maximum artifact duration is scaled, other parameters may be additionally scaled by similar amounts, such as the times between the Onset, Pre-Peak, Trigger, Trough, Post-Peak, and Post-Baseline of FIG. 7A or the times between the Onset, Trigger, Trough, Post-Peak, and Post-Baseline of FIG. 7B.

In some embodiments, the maximum duration of an artifact detection area is bounded. In such embodiments, two factors may bound the maximum duration. The first factor may be limits on the detection range for morphological elements and the second factor may be the estimated artifact duration based on peak-to-peak amplitude.

In some embodiments, the estimated duration for positive form artifacts is explicitly bounded by the maximum estimated duration for a positive form artifact (e.g. 15000ms). Additionally, the maximum estimated duration may be applied to artifacts larger than a threshold amplitude. In some examples, the threshold may be calculated using EQ. 1 wherein Duration Artifact is 15000ms. However other thresholds may be used.

FIG. 7A provides an exemplary maximum duration for a positive form artifact. As shown, the maximum duration of a positive form artifact may be 15000ms, however other values above and below 15000ms have been contemplated.

FIG. 7B provides an exemplary maximum duration for a negative form artifact. As shown, the maximum duration of various artifacts (e.g. positive form and negative form) need not be the same. For example, FIG. 7B provides the maximum duration for a negative form artifact as 12692ms. However, 12692ms is only an exemplary value for the maximum duration for a negative form artifact, values above and below 12692ms. Similarly, the maximum duration for a negative form artifact can be the same or different than the maximum duration for a positive form artifact.

In some embodiments, the estimated duration for negative form artifacts is explicitly bounded by the maximum estimated duration for a positive form artifact (e.g. 12692ms). Additionally, the maximum estimated duration may be applied to artifacts larger than a threshold amplitude. In some examples, the threshold may be calculated using EQ. 2 wherein Duration Artifact is 12692ms. However other thresholds may be used.

In some embodiments, when an artifact (e.g. positive form artifact, negative form artifact, or the like) is found, they are omitted from the data sample set. In such examples, the data within the artifact may be interpolated using the data from each side of the artifact. Alternatively, the artifact may be simply marked in such a way to notify a user (e.g. a physician) that the data in that window of time is an artifact rather than diagnostic information.

Additionally or alternatively, an aggressive Urocap Noise Detection (AUND) method may be used as an aggressive noise detector. The AUND method may also operate on the volume channel of a uroflowmeter (e.g. uroflowmeter 110) similar to the noise artifact detection method 300 described herein. In some embodiments, the AUND method can be used to remove a wider range of artifacts. In such embodiments, the AUND method may be used during times where no leak is present, such as times other than near active flows or leaks. However, the AUND method may be used at other times, such as near active flow or leaks. In some embodiments, the AUND method may be used separately from the noise artifact detection method as described herein. Additionally or alternatively, the AUND method may be used tandem. Furthermore, the AUND method as discussed herein may be performing using an AUND system, such as a system incorporated into the uroflowmetry system 100 as shown in FIG. 1.

The AUND method may be used to detect period of time, or groupings of consecutively collected sample volume data, called baselines. A baseline, as described herein, may be an interval where there is high confidence in the estimated expected volume value, such as when the uroflowmeter is in a steady state. In some embodiments, if there is an interval between two baselines, and wherein the respective baseline volume values are close enough together such that no flow or leak of significance could have occurred all value changes within the interval between the two baselines can be considered noise. When a portion of the data is identified as noise, said portion of the data may be removed, interpolated, marked, or the like. For example, a FlowNoiseDataRetraction Event can then be generated on that interval to remove all noise and artifacts within. Additionally or alternatively, the data within the interval may be interpolated using any method known to one of ordinary skill in the art.

In some embodiments, the AUND method may consider any intervals within a threshold and any baseline differences within a threshold. In some embodiments, the threshold for the intervals is an interval which is 30 seconds or less; however other intervals may be used, such as more than 30 seconds or less than 30 seconds. Alternatively, a high and low threshold for the intervals may be used, such as intervals between 1 second and 30 seconds. Similarly, the baseline threshold may be values less than 0.4 mL; however other values may be used, such as more than 0.4 mL or less than 0.4 mL. Alternatively, a high and low threshold may be used for the baseline difference, such as values between 0.1 and 0.4 mL.

In some embodiments, the AUND method may be used in real time, such as while sample data is being collected or shortly after (e.g. 1/10th of a second, a predetermined amount of samples, as soon as data is gathered in the buffer, as soon as a threshold is detected, etc.). Alternatively, the AUND method may happen after data collection, such as after a predetermine amount of time after a threshold, upon user input, or after all samples have been collected.

Various algorithms and/or methods may be used to determine baselines within the sample data and the confidence for those baselines. Two exemplary types of baselines which can be used are temporary baselines and global baselines.

Temporary baselines can be used to remove clearly defined artifacts with low latency as opposed to waiting for other methods which may depend on additional samples to define a baseline. For example, if an unstable period in volume sample data is detected, temporary baselines may be used to determine whether or not the unstable period contains a flow, leak, or is an artifact.

Global baselines can be calculated using larger sample intervals when compared to the temporary baselines. As a result, global baselines can have a higher confidence in estimating the baseline values, which may allow for better analysis of whether or not the unstable period in the volume sample contains a flow, leak, or is an artifact. Furthermore, by using more samples, it may be possible to use additional checks to determine if a baseline exists or not.

The mean of an interval can be calculated representing the line of best fit that can be plotted using the samples with the restriction of having a slope of 0. The mean squared error may measure how close the samples within an interval are with respect to the mean. Lower mean squared error values can indicate a higher confidence of the mean value representing the sample interval.

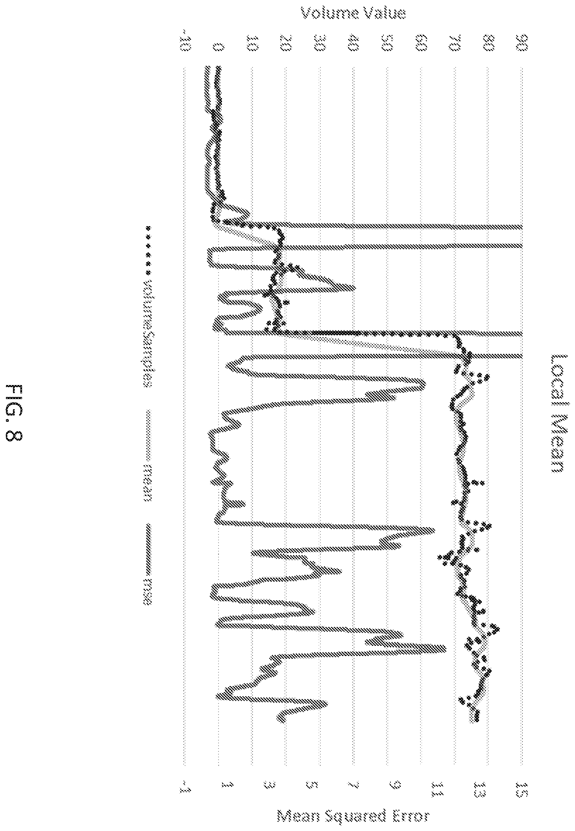

FIG. 8 provides an example showing the relationship between a mean (blue line), the mean squared error [1/confidence] (gray line), and the volume samples (purple line). In some embodiments, significant value changes in the mean with low mean squared error can indicate a flow or leak while means with high mean squared error may indicate that the differences are due to noise or an artifact.

Calculating the $R^2$ value may give an idea of how much correlation exists within the sample interval. In some embodiments, a high $R^2$ value may indicate that the volume samples within the interval are trending up or down with a high degree of confidence. In embodiments comprising a high $R^2$ value, a proper baseline may not be able to be established as the uroflowmeter may not be in a steady state. In contrast, if the $R^2$ value is low, then a proper baseline may be able to be established, as the uroflowmeter may be in a steady state.

In embodiments having more samples, it may also be possible to perform more rigorous calculations when determining the potential baseline value. Rather than using the mean of the sample interval as the baseline value and the mean squared average as the confidence, it may also be possible to use the cluster based median of the sample interval. In such embodiments, samples within the interval may be grouped into value clusters limited by a predefined value for the range of values allowed in each cluster. Then, the baseline reference may be given by the median of samples within the cluster that contains the largest number of volume samples. The confidence of this value may be measured by the percentage of samples in the largest cluster versus the total number of samples analyzed within the interval. As such, a high cluster size percentage can indicate a higher confidence.

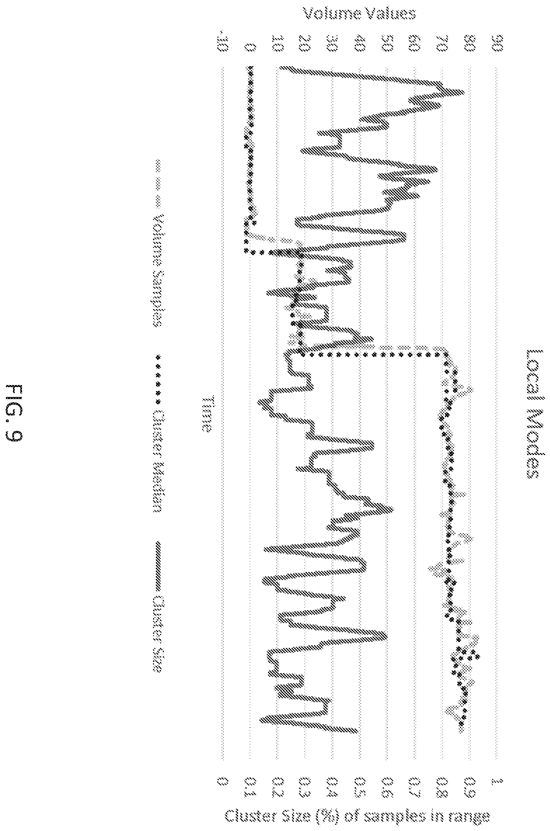

FIG. 9 details an exemplary relationship between the cluster median (orange line), cluster size [confidence] (green line), and the volume samples (blue line). As described herein, value changes in the cluster median with high confidence may indicate that a flow or leak while low confidence may indicate noise or an artifact.

A threshold value may be configured for combinations of ambiguous regions between each of the baselines found, as described herein. For example, between two global baselines, between two temporary baselines, between a global baseline and a temporary baseline, or between any two other baselines known to one of ordinary skill in the art. Then as discussed herein, if the change between the baseline values is larger than the threshold value, then the interval is may be considered to be a leak or flow; otherwise, if the change between the baseline values is smaller than the predetermined threshold value, the interval may be considered an artifact and the data comprised within the interval may be removed, interpolated, marked, or the like. For example, a FlowNoiseDataRetraction artifact can be generated or any other interpolation method known to one of ordinary skill in the art.

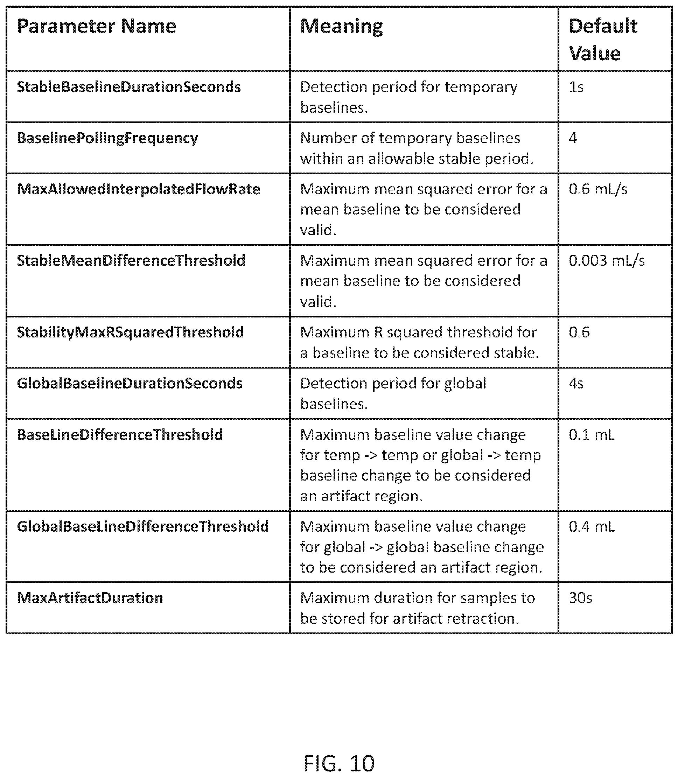

FIG. 10 provides some exemplary values when performing baseline checks and/or determining whether or not an interval between two baselines is a flow, a leak, noise, or an artifact. In some embodiments, the values in FIG. 10 are not intended to be adjusted by a user, however in other embodiments, the values may be adjustable, such as by a user interface. Furthermore, the values shown in FIG. 10 detail a single embodiment and are no way limiting. Values above and below the values shown in FIG. 10 have been contemplated.

Various embodiments have been described. Such examples are non-limiting, and do not define or limit the scope of the invention, which is defined by the appended claims.

## Claims

1. A uroflowmetry system (100) for analyzing uroflowmeter data, comprising:

   a uroflowmeter device (110), the uroflowmeter device configured to produce volume sample data representative of a volume of liquid (125) within the uroflowmetry system; and
   an external computation device (150), the external computation device being configured to:

      receive volume sample data from the uroflowmeter device, the device being **characterized by** being configured to
      calculate the slope of the volume sample data; and
      if the calculated slope reaches a trigger threshold:

         determining if an artifact is present in the volume sample data, including

            comparing the morphology of the potential artifact to morphologies of known artifacts, and
            comparing the value of the volume sample data before and after the potential artifact; and

         if an artifact is determined to be present in the volume sample data and the value of

            the volume sample data before the detected artifact is less than or equal to
            the volume sample data after the detected artifact, remove the portion of
            the volume sample data which represents the detected artifact.

2. The uroflowmetry system (100) of claim 1, wherein the uroflowmeter device (110) comprises a uroflowmeter sensor.

3. The uroflowmetry system of claim 1 or 2, wherein the external computation device comprises at least one of a computer, a tablet, and a smartphone.

4. A method of providing uroflowmeter data, the method comprising:

   receiving volume sample data representative of volume sample data from a uroflowmeter device;
   the method being **characterized by** calculating the slope of the volume sample data; and
   if the calculated slope reaches a trigger threshold:

      determining if an artifact is present in the volume sample data, including

         comparing the morphology of the potential artifact to morphologies of known artifacts, and
         comparing the value of the volume sample data before and after the potential artifact; and

      if an artifact is determined to be present in the volume sample data and the value of the

         volume sample data before the determined artifact is less than or equal to the
         volume sample data after the determined artifact, removing the portion of the volume
         sample data which represents the detected artifact.

5. The system (200) of any one of claims 1 to 3, or the method of claim 4, wherein calculating the slope of the volume sample data comprises:

calculating an average slope; and
adjusting the slope by the average slope and wherein calculating the average slope preferably comprises using a least-squares best fit model.

6. The system (200) of any one of claims 1 to 3 or of claim 5, or the method of claim 4 or 5, wherein the trigger threshold comprises a lower slope value.

7. The system (200) or method of claim 6, wherein the trigger threshold comprises when the calculated slope is at or below 0 mL/s.

8. The system (200) of any one of claims 1 to 3, or of claims 5 to 7, or the method of any of claims 4 to 7, wherein an artifact comprises volumetric data comprising an event, wherein the event comprises at least one of: a door opening, a door closing, an HVAC system running, footsteps, and mechanical vibrations

9. The system (200) of any one of claims 1 to 3, or claims 5 to 8, or the method of any of claims 4 to 8, wherein receiving volume sample data comprises receiving a plurality of volume sample data points, for example wherein receiving the plurality of volume sample data points further comprises receiving the volume sample data points from a rolling buffer.

10. The system (200) of any one of claims 1 to 3, or of claims 5 to 9, or the method of any of claims 4 to 9, wherein removing the portion of volume sample data which represents the detected artifact comprises interpolating volume sample data from each side of the detected artifact.

11. The system (200) of any one of claims 1 to 3, or of claims 5 to 10, or the method of any one of claims 4 to 10, further comprising applying a bandpass filter prior to calculating the slope of the volume sample data.

12. The system (200) or method of claim 11, wherein the bandpass filter is between 0.4Hz and 0.8Hz.

13. The system (200) of any one of claims 1 to 3, or of claims 5 to 12, or the method of any of claims 4 to 12, wherein determining if an artifact is present in the volume sample data comprises:

   determining a trigger, wherein the trigger is the point when the trigger threshold is reached;
   determining a baseline, wherein the baseline is an area prior to the start of the potential artifact;
   determining a post-baseline, wherein the post-baseline is an area after the end of the potential artifact;
   determining a trough, wherein the trough is the lowest local minima bounded by the trigger and time after the event;
   determining a post peak, wherein the post-peak is the largest local maxima bounded by the trigger and the post-baseline;
   determining an onset, wherein determining the onset comprises at least one of:

   locating a local minima or flat area in the span prior from the pre-peak that is less than 10% of the pre-peak amplitude from the baseline, wherein the located local minima or flat area is the onset, or
   locating a first point which is the steepest positive slope between the baseline and the trigger, then locating a second point which is the flattest point between the first point and the baseline, wherein the second point is the onset.

14. The system (200) or method of claim 13 wherein:

   if the pre-peak is greater than the baseline and the post peak, the artifact is identified as a positive form artifact; and
   if the trough is after the trigger, the artifact is identified as a negative form artifact.

15. The system (200) or method of claim 13 or claim 14, further comprising:

   determining a baseline delta, wherein the baseline delta is the difference between the baseline and the post-baseline; and
   if the baseline delta is above a certain value, then the potential artifact is determined to not be an artifact, and wherein the method preferably comprises
   determining a peak-to-trough amplitude, wherein the peak-to-trough amplitude is the difference between the lowest and highest value between the baseline and the post-baseline;
comparing the peak-to-trough amplitude to the baseline delta; and
if the baseline delta value is higher than 15% of the peak-to-trough amplitude, determine that the potential artifact is not an artifact.

**Patentansprüche**

1. Harnflussmesssystem (100) zum Analysieren von Harnflussmessdaten, das Folgendes umfasst:

   eine Harnflussmessvorrichtung (110), wobei die Harnflussmessvorrichtung zum Erzeugen von Volumenprobendaten, die für ein Flüssigkeitsvolumen (125) in dem Harnflussmesssystem repräsentativ sind, konfiguriert ist; und
   eine externe Rechenvorrichtung (150), wobei die externe Rechenvorrichtung konfiguriert ist zum:

   Empfangen von Volumenprobendaten von der Harnflussmessvorrichtung, wobei die Vorrichtung **dadurch gekennzeichnet ist, dass** sie konfiguriert ist zum
   Berechnen der Steigung der Volumenprobendaten; und,
   wenn die berechnete Steigung eine Auslöseschwelle erreicht:

   Ermitteln, ob in den Volumenprobendaten ein Artefakt vorhanden ist, einschließlich

   Vergleichen der Morphologie des potentiellen Artefakts mit Morphologien bekannter Artefakte und Vergleichen des Werts der Volumenprobendaten vor und nach den potentiellen Artefakten; und,

   wenn ermittelt wird, dass ein Artefakt in den Volumenprobendaten vorhanden ist, und der Wert der Volumenprobendaten vor dem erkannten Artefakt kleiner als die oder gleich den Volumenprobendaten nach dem erkannten Artefakt ist, Entfernen des Teils der Volumenprobendaten, der den erkannten Artefakt repräsentiert.

2. Harnflussmesssystem (100) nach Anspruch 1, wobei die Harnflussmessvorrichtung (110) einen Harnflussmesssensor umfasst.

3. Harnflussmesssystem nach Anspruch 1 oder 2, wobei die externe Rechenvorrichtung wenigstens einen von einem Computer, einem Tablet und einem Smartphone umfasst.

4. Verfahren zum Bereitstellen von Harnflussmessdaten, wobei das Verfahren Folgendes umfasst:

   Empfangen von Volumenprobendaten, die für Volumenprobendaten von einer Harnflussmessvorrichtung repräsentativ sind;
   wobei das Verfahren **gekennzeichnet ist durch** Berechnen der Steigung der Volumenprobendaten; und,
   wenn die berechnete Steigung eine Auslöseschwelle erreicht:

   Ermitteln, ob in den Volumenprobendaten ein Artefakt vorhanden ist, einschließlich

   Vergleichen der Morphologie des potentiellen Artefakts mit Morphologien bekannter Artefakte und Vergleichen des Werts der Volumenprobendaten vor und nach dem potentiellen Artefakt; und,

   wenn ermittelt wird, dass ein Artefakt in den Volumenprobendaten vorhanden ist und der Wert der Volumenprobendaten vor dem ermittelten Artefakt kleiner als die oder gleich den Volumenprobendaten nach dem erkannten Artefakt ist, Entfernen des Teils der Volumenprobendaten, der den erkannten Artefakt repräsentiert.

5. System (200) nach einem der Ansprüche 1 bis 3 oder Verfahren nach Anspruch 4, wobei das Berechnen der Steigung der Volumenprobendaten Folgendes umfasst:

   Berechnen einer Durchschnittssteigung; und
   Anpassen der Steigung um die Durchschnittssteigung und wobei das Berechnen der Durchschnittssteigung vorzugsweise das Verwenden eines Modells der besten Anpassung nach der Methode der kleinsten Quadrate umfasst.

6. System (200) nach einem der Ansprüche 1 bis 3 oder Anspruch 5 oder Verfahren nach Anspruch 4 oder 5, wobei die Auslöseschwelle einen niedrigeren Steigungswert umfasst.

7. System (200) oder Verfahren nach Anspruch 6, wobei die Auslöseschwelle umfasst, dass die berechnete Steigung auf oder unter 0 ml/s ist.

8. System (200) nach einem der Ansprüche 1 bis 3 oder der Ansprüche 5 bis 7 oder Verfahren nach einem der Ansprüche 4 bis 7, wobei ein Artefakt volumetrische Daten umfasst, die ein Ereignis umfassen, wobei das Ereignis wenigstens eines der folgenden umfasst: Öffnen einer Tür, Schließen einer Tür, ein in Betrieb befindliches HLK-System, Schritte und mechanische Vibrationen.

9. System (200) nach einem der Ansprüche 1 bis 3 oder der Ansprüche 5 bis 8 oder Verfahren nach einem der Ansprüche 4 bis 8, wobei das Empfangen von Volumenprobendaten das Empfangen einer Vielzahl von Volumenprobendatenpunkten umfasst, beispielsweise wobei das Empfangen der Vielzahl von Volumenprobendatenpunkten ferner das Empfangen der Volumenprobendatenpunkte aus einem Ringpuffer umfasst.

10. System (200) nach einem der Ansprüche 1 bis 3 oder der Ansprüche 5 bis 9 oder Verfahren nach einem der Ansprüche 4 bis 9, wobei das Entfernen des Teils der Volumenprobendaten, der den erkannten Artefakt repräsentiert, das Interpolieren von Volumenprobendaten von jeder Seite des erkannten Artefakts umfasst.

11. System (200) nach einem der Ansprüche 1 bis 3 oder der Ansprüche 5 bis 10 oder Verfahren nach einem der Ansprüche 4 bis 10, das ferner das Anwenden eines Bandpassfilters vor Berechnen der Steigung der Volumenprobendaten umfasst.

12. System (200) oder Verfahren nach Anspruch 11, wobei das Bandpassfilter zwischen 0,4 Hz und 0,8 Hz ist.

13. System (200) nach einem der Ansprüche 1 bis 3 oder der Ansprüche 5 bis 12 oder Verfahren nach einem der Ansprüche 4 bis 12, wobei das Ermitteln, ob ein Artefakt in den Volumenprobendaten vorhanden ist, Folgendes umfasst:

Ermitteln eines Auslösers, wobei der Auslöser der Punkt ist, an dem die Auslöseschwelle erreicht wird;
Ermitteln einer Basislinie, wobei die Basislinie ein Bereich vor Anfang des potentiellen Artefakts ist;
Ermitteln einer Nach-Basislinie, wobei die Nach-Basislinie ein Bereich nach dem Ende des potentiellen Artefakts ist;
Ermitteln eines Tiefpunkts, wobei der Tiefpunkt die niedrigsten lokalen Minima ist, die von dem Auslöser und der Zeit nach dem Ereignis umgrenzt sind;
Ermitteln eines Nach-Hochpunkts, wobei der Nach-Hochpunkt die höchsten lokalen Maxima ist, die von dem Auslöser und der Nach-Basislinie umgrenzt sind;
Ermitteln eines Beginns, wobei das Ermitteln des Beginns zumindest eines der folgenden umfasst:

Ausmachen (eines) lokaler Minima oder flachen Bereichs in der Spanne vor dem Vor-Hochpunkt, die kleiner als 10 % der Vor-Hochpunkt-Amplitude von der Basislinie sind/ist, wobei die bzw. der ausgemachte(n) lokale(n) Minima oder flache Bereich der Beginn ist, oder
Ausmachen eines ersten Punkts, der die steilste positive Steigung zwischen der Basislinie und dem Auslöser ist, dann Ausmachen eines zweiten Punkts, der der flachste Punkt zwischen dem ersten Punkt und der Basislinie ist, wobei der zweite Punkt der Beginn ist.

14. System (200) oder Verfahren nach Anspruch 13, wobei:

wenn der Vor-Hochpunkt größer als die Basislinie und der Nach-Hochpunkt ist, der Artefakt als ein Artefakt positiver Form identifiziert wird; und,
wenn der Tiefpunkt nach dem Auslöser ist, der Artefakt als ein Artefakt negativer Form identifiziert wird.

15. System (200) oder Verfahren nach Anspruch 13 oder Anspruch 14, ferner umfassend:

Ermitteln eines Basislinien-Deltawerts, wobei der Basislinien-Deltawert die Differenz zwischen der Basislinie und der Nach-Basislinie ist; und,

wenn der Basislinien-Deltawert über einem gewissen Wert ist, dann wird ermittelt, dass der potentielle Artefakt kein Artefakt ist, und wobei das Verfahren vorzugsweise das Ermitteln einer Hochpunkt-zu-Tiefpunkt-Amplitude umfasst, wobei die Hochpunkt-zu-Tiefpunkt-Amplitude die Differenz zwischen dem niedrigsten und dem höchsten Wert zwischen der Basislinie und der Nach-Basislinie ist;

Vergleichen der Hochpunkt-zu-Tiefpunkt-Amplitude mit dem Basislinien-Deltawert; und,

wenn der Basislinien-Deltawert höher als 15 % der Hochpunkt-zu-Tiefpunkt-Amplitude ist, Ermitteln, dass der potentielle Artefakt kein Artefakt ist.

**Revendications**

1. Système d'urodébitmétrie (100) pour analyser des données d'urodébitmètre, comprenant :

   un dispositif urodébitmètre (110), le dispositif urodébitmètre étant configuré pour produire des données d'échantillon de volume représentatives d'un volume de liquide (125) dans le système d'urodébitmétrie ; et
   un dispositif de calcul externe (150), le dispositif de calcul externe étant configuré pour :
   recevoir des données d'échantillon de volume du dispositif urodébitmètre, le dispositif étant caractérisé en étant configuré pour :

      calculer la pente des données d'échantillon de volume ; et
      si la pente calculée atteint un seuil de déclenchement :
      déterminer si un artefact est présent dans les données d'échantillon de volume, comprenant

         comparer la morphologie de l'artefact potentiel aux morphologies d'artefacts connus, et
         comparer la valeur des données d'échantillon de volume avant et après l'artefact potentiel ; et

      s'il est déterminé qu'un artefact est présent dans les données d'échantillon de volume et que la valeur des données d'échantillon de volume avant la détection de l'artefact est moindre ou égale aux données d'échantillon de volume après la détection de l'artefact, retirer la partie des données d'échantillon de volume qui représente l'artefact détecté.

2. Système d'urodébitmétrie (100) selon la revendication 1, dans lequel le dispositif urodébitmètre (110) comprend un capteur d'urodébitmètre.

3. Système d'urodébitmétrie selon la revendication 1 ou 2, dans lequel le dispositif de calcul externe comprend au moins l'un d'entre un ordinateur, une tablette et un téléphone intelligent.

4. Procédé de fourniture de données d'urodébitmètre, le procédé comprenant :

   recevoir des données d'échantillon de volume représentatives de données d'échantillon de volume d'un dispositif urodébitmètre ;
   le procédé étant caractérisé en calculant la pente des données d'échantillon de volume ; et
   si la pente calculée atteint un seuil de déclenchement :

      déterminer si un artefact est présent dans les données d'échantillon de volume, comprenant

         comparer la morphologie de l'artefact potentiel à des morphologies d'artefacts connus, et
         comparer la valeur des données d'échantillon de volume avant et après l'artefact potentiel ; et

      s'il est déterminé qu'un artefact est présent dans les données d'échantillon de volume et que la valeur des données d'échantillon de volume avant que l'artefact ne soit déterminé est moindre ou égale aux données d'échantillon de volume après que l'artefact a été déterminé, retirer la partie des données d'échantillon de volume qui représente l'artefact détecté.

5. Système (200) selon l'une quelconque des revendications 1 à 3, ou procédé selon la revendication 4, dans lesquels calculer la pente des données d'échantillon de volume comprend :

calculer une pente moyenne ; et

ajuster la pente par la pente moyenne et dans lesquels calculer la pente moyenne comprend de préférence utiliser un modèle d'ajustement par régression des moindres carrés.

6. Système (200) selon l'une quelconque des revendications 1 à 3 ou la revendication 5, ou le procédé selon la revendication 4 ou 5, dans lesquels le seuil de déclenchement comprend une valeur de pente inférieure.

7. Système (200) ou procédé selon la revendication 6, dans lesquels le seuil de déclenchement comprend lorsque la pente calculée est à ou au-dessous de 0 ml/s.

8. Système (200) selon l'une quelconque des revendications 1 à 3, ou selon les revendications 5 à 7, ou procédé selon l'une quelconque des revendications 4 à 7, dans lesquels un artefact comprend des données volumétriques comprenant un événement, dans lesquels l'événement comprend au moins l'un d'entre : ouvrir une porte, fermer une porte, un système CVC en marche, des pas et des vibrations mécaniques.

9. Système (200) selon l'une quelconque des revendications 1 à 3, ou selon les revendications 5 à 8, ou procédé selon l'une quelconque des revendications 4 à 8, dans lesquels recevoir des données d'échantillon de volume comprend recevoir une pluralité de points de données d'échantillon de volume, par exemple dans lesquels recevoir la pluralité de points de données d'échantillon de volume comprend recevoir les points de données d'échantillon de volume d'un tampon en défilement.

10. Système (200) selon l'une quelconque des revendications 1 à 3, ou selon les revendications 5 à 9, ou procédé selon l'une quelconque des revendications 4 à 9, dans lesquels retirer la partie des données d'échantillon de volume qui représente l'artefact détecté comprend interpoler des données d'échantillon de volume de chaque côté de l'artefact détecté.

11. Système (200) selon l'une quelconque des revendications 1 à 3, ou selon les revendications 5 à 10, ou procédé selon l'une quelconque des revendications 4 à 10, comprenant en outre appliquer un filtre passe-bande avant de calculer la pente des données d'échantillon de volume.

12. Système (200) ou procédé selon la revendication 11, dans lesquels le filtre passe-bande est d'entre 0,4 Hz et 0,8 Hz.

13. Système (200) selon l'une quelconque des revendications 1 à 3, ou selon les revendications 5 à 12, ou procédé selon l'une quelconque des revendications 4 à 12, dans lesquels déterminer si un artefact est présent dans les données d'échantillon de volume comprend :

déterminer un déclenchement, dans lesquels le déclenchement est le point auquel le seuil de déclenchement est atteint ;

déterminer une ligne de base, dans lesquels la ligne de base est une zone précédant le début de l'artefact potentiel ;

déterminer une post-ligne de base, dans lesquels la post-ligne de base est une zone après la fin de l'artefact potentiel ;

déterminer un creux, dans lesquels le creux est le minimum local le plus bas délimité par le déclenchement et le temps après l'événement ;

déterminer un post-pic, dans lesquels le post-pic est le maximum local le plus grand délimité par le déclenchement et la post-ligne de base ;

déterminer un démarrage, dans lesquels déterminer le démarrage comprend au moins l'un d'entre :

localiser un minimum local ou une zone plate dans l'étendue préalablement au pré-pic qui est de moins de 10 % de l'amplitude pré-pic à partir de la ligne de base, dans lesquels le minimum local ou zone plate localisé est le démarrage, ou

localiser un premier point qui est la pente positive la plus raide entre la ligne de base et le déclenchement, puis localiser un deuxième point qui est le point le plus plat entre le premier point et la ligne de base, dans lesquels le deuxième point est le démarrage.

14. Système (200) ou procédé selon la revendication 13, dans lesquels :

si le pré-pic est plus grand que la ligne de base et le post-pic, l'artefact est identifié comme un artefact de forme positive ; et

si le creux survient après le déclenchement, l'artefact est identifié comme un artefact de forme négative.

15. Système (200) ou procédé selon la revendication 13 ou la revendication 14, comprenant en outre :

déterminer un delta de ligne de base, dans lesquels le delta de ligne de base est la différence entre la ligne de base et la post-ligne de base ; et

si le delta de ligne de base est au-dessus d'une certaine valeur, alors l'artefact potentiel est déterminé ne pas être un artefact, et dans lesquels le procédé comprend de préférence

déterminer une amplitude de pic-à-creux, dans lesquels l'amplitude de pic-à-creux est la différence entre la valeur la plus basse et la plus haute entre la ligne de base et la post-ligne de base ;

comparer l'amplitude de pic-à-creux au delta de ligne de base ; et

si la valeur du delta de ligne de base dépasse 15 % de l'amplitude de pic-à-creux, déterminer que l'artefact potentiel n'est pas un artefact.

FIG. 1

Positive Form

FIG. 2A

Negative Form

FIG. 2B

FIG. 3

FIG. 4A

FIG. 4B

FIG. 4A

FIG. 4B

FIG. 5

| Parameter Name | Meaning | Default Value |
|---|---|---|
| FlowArtifactBandStartHz | Corner frequency of Lower bounds of the artifact band. Frequencies below this will have a smaller effect on the detection. | 0.07 Hz |
| FlowArtifactBandEndHz | Corner frequency of Upper bounds of the artifact band. Frequencies above this will have a smaller effect on the detection. | 0.6 Hz |
| FlowArtifactBandPassOrder | Filter Order. Specifies the steepness of roll-off after corner frequencies. | 64 |
| FlowArtifactMaximumDetectionDuration | Maximum window over which the algorithm will look for artifact forms. | 15000ms |
| FlowArtifactTriggerPerSecondSlopeIdentifier | Minimum negative slope required to cause trigger. | -0.32 mL/s |
| FlowArtifactTriggerSlopeWindow | Length of best-fit window for slope calculation. | 42 Samples |
| FlowArtifactPeakToPeakMinimum | Minimum peak-to-peak amplitude required for artifact detection. | 0.08 mL |
| FlowArtifactHoldOffTimeMS | Time between potential artifact complexes. | 10ms |
| FlowArtifactMinPeakFlow | Minimum Peak flow for artifact detection. | 0.48 mL/s |

FIG. 6

FIG. 7A

FIG. 7B

FIG. 9

| Parameter Name | Meaning | Default Value |
|---|---|---|
| StableBaselineDurationSeconds | Detection period for temporary baselines. | 1s |
| BaselinePollingFrequency | Number of temporary baselines within an allowable stable period. | 4 |
| MaxAllowedInterpolatedFlowRate | Maximum mean squared error for a mean baseline to be considered valid. | 0.6 mL/s |
| StableMeanDifferenceThreshold | Maximum mean squared error for a mean baseline to be considered valid. | 0.003 mL/s |
| StabilityMaxRSquaredThreshold | Maximum R squared threshold for a baseline to be considered stable. | 0.6 |
| GlobalBaselineDurationSeconds | Detection period for global baselines. | 4s |
| BaseLineDifferenceThreshold | Maximum baseline value change for temp -> temp or global -> temp baseline change to be considered an artifact region. | 0.1 mL |
| GlobalBaseLineDifferenceThreshold | Maximum baseline value change for global -> global baseline change to be considered an artifact region. | 0.4 mL |
| MaxArtifactDuration | Maximum duration for samples to be stored for artifact retraction. | 30s |

FIG. 10

**REFERENCES CITED IN THE DESCRIPTION**

**Patent documents cited in the description**

- US 2006235353 A1